(19) Europäisches Patentamt

European Patent Office

Office européen des brevets

(11) Veröffentlichungsnummer: **0 147 819 B1**

(12)

# EUROPÄISCHE PATENTSCHRIFT

(45) Veröffentlichungstag der Patentschrift: **03.06.92**

(21) Anmeldenummer: **84116054.2**

(22) Anmeldetag: **21.12.84**

(51) Int. Cl.⁵: **C12P 21/02**, C12N 15/70, A61K 37/02

(54) Reinigung von rekombinanten Interleukin-2.

(30) Priorität: **23.12.83 US 564986**

(43) Veröffentlichungstag der Anmeldung:
**10.07.85 Patentblatt 85/28**

(45) Bekanntmachung des Hinweises auf die
Patenterteilung:
**03.06.92 Patentblatt 92/23**

(84) Benannte Vertragsstaaten:
**AT BE CH DE FR GB IT LI LU NL SE**

(56) Entgegenhaltungen:
**EP-A- 0 091 539      EP-A- 0 092 163**
**EP-A- 0 094 317      EP-A- 0 145 390**
**EP-A- 0 156 373      EP-A- 0 163 249**

(73) Patentinhaber: **F. HOFFMANN-LA ROCHE AG**
**Postfach 3255**
**CH-4002 Basel(CH)**

(72) Erfinder: **Kung, Hsiang-Fu**
**21 West Lincolnstreet**
**Verona, N.J. 07044(US)**
Erfinder: **Yamazaki, Shigeko**
**58 Johnson Street**
**Clifton, N.J. 07014(US)**

(74) Vertreter: **Lederer, Franz, Dr. et al**
**Lederer, Keller & Riederer Patentanwälte**
**Lucile-Grahn-Strasse 22**
**W-8000 München 80(DE)**

## Beschreibung

Human-Interleukin-2 (IL-2), in der Vergangenheit als T-Zellen Wachstumsfaktor beschrieben, ist ein lösliches Protein, welches von Lektin- oder Antigen-aktivierten T-Zellen synthetisiert werden kann und das in der Lage ist die Lymphozyten-Reaktivität zu modulieren und die Langzeit-in vitro-Kultur Antigen-spezifischer T-Effektor-Lymphozyten zu fördern. IL-2 verstärkt nachgewiesenermassen auch die Thymozyten-Mitogenese und induziert cytotoxische T-Lymphozyten-Reaktivität. Somit ist diese Lymphozyten-regulierende Substanz für die Steigerung der humoralen und zellgebundenen Immunantwort und zur Wiederherstellung einer normalen humoralen und zellgebundenen Immunität nützlich. Diese nachgewiesenen immunologischen Aktivitäten zeigen, dass IL-2 zur medizinischen Immuntherapie von Krankheiten des Immunsystems einschliesslich neoplastischer Krankheiten, bakterieller und viraler Infektionen, Immunmangelkrankheiten, Autoimmunkrankheiten usw. eingesetzt werden kann (Papermaster, B. et al., Adv. Immunopharm., 507, [1980]). Ein kürzlich erschienener Uebersichtsartikel im Journal of The American Medical Association, Vol. 249, No. 2 (14. Januar 1983) bespricht auf den Seiten 166-171 die klinische Applikation verschiedener Lymphokine unter besonderer Berücksichtigung des Human-IL-2.

Die Reinigung von homogenem Human-IL-2 aus induzierten menschlichen malignen Zellen bis zur Homogenität wurde vor kurzem durch ein mehrstufiges hochauflösendes Flüssigkeitschromatographie-Verfahren (HPLC) erreicht. Das resultierende homogene Human-IL-2 war gekennzeichnet durch eine spezifische Aktivität von etwa 1.4 x 10$^9$ E/mg (siehe z.B. Europäische Patentanmeldung Nr. 83.109202.8, Publikationsnummer 106 179)

Taniguchi et al. berichteten über die Klonierung und Sequenzierung des IL-2 Gens und die Expression von unreifen Human-IL-2 auf dem "Third Annual Recombinant DNA Congress" in Philadelphia. Pennsylvania, am 9. Februar 1983 (Nature, 302, 305-310 [1983]). Die cDNA, von der sich die Aminosäuresequenz des IL-2 Proteins ableitet, wurde aus mRNA von Jurkat Zellen, die mit Concanavalin A angeregt worden waren, isoliert. Der cDNA Klon mit voller Länge ist 800 Basenpaare lang und codiert für ein Protein mit 153 Aminosäuren. Siehe auch Europäische Patentanmeldung Publikations-Nr. 91539.

Auch von anderen Arbeitsgruppen wurde die Klonierung und Sequenzierung des IL-2 Gens beschrieben. Siehe Devos et al., "Molecular Cloning of Human Interleukin-2 cDNA and Its Expression in E. coli", Nucleic Acids Research, 11, 4307--4323 (1983). Siehe auch Europäische Patentanmeldung Publikations-Nr. 118 977.

Obwohl die Expression von IL-2 in transformierten Wirtszellen, hauptsächlich in E. coli, in verschiedenen Literaturstellen beschrieben ist, werden keine Reinigungsverfahren offenbart, die es dem Fachmann erlauben würden, reifes rekombinantes Human IL-2 in homogener oder im wesentlichen reiner Form zu erhalten.

Die vorliegende Erfindung betrifft ein Verfahren zur Reinigung von rekombinantem, insbesondere reifem Human-IL-2. Sie beschreibt ein Verfahren zur Reinigung von rekombinantem reifem Human-IL-2 bis zur Homogenität, das über die Methoden des Standes der Technik hinausgeht und das dadurch gekennzeichnet ist, dass man

(a) Mikroorganismen, die mit einer DNA-Sequenz transformiert sind, die für reifes Human-IL-2 kodiert, kultiviert;

(b) die transformierten Mikroorganismen zur Expression und Akkumulation von reifem Human-IL-2 veranlasst;

(c) die Mikroorganismen lysiert, wobei ein Zellysat erhalten wird;

(d) die im Zellysat enthaltenen Zellmembranbestandteile abtrennt;

(e) aus den isolierten Zellmembranbestandteilen IL-2 mittels einer Waschlösung, die etwa 4 bis 7M Guanidin-HCl enthält, extrahiert; und

(f) das IL-2 aus der Waschlösung chromatographisch reinigt.

EP-A-0 145 390 beschreibt zwar ein Verfahren zur Herstellung von rekombinantem Human-IL-2 mit Hilfe von 7M Guanidin-HCl und Chromatographie, jedoch wird bei diesem Verfahren eine vorhergehende Lyse der Mikroorganismen und die Isolierung von Membranbestandteilen nicht durchgeführt. Die spezifische Aktivität des gewonnenen IL-2 ist niedriger als diejenige des IL-2, das gemäss dem Verfahren erhalten wurde, das in der vorliegenden Anmeldung beschrieben ist.

### Kurze Beschreibung der Figuren

Figur 1 gibt die DNA Nukleotidsequenz von pIL-2-2B und die entsprechende Aminosäuresequenz von reifem Human-IL-2 wieder. Das aminoterminale Ende von reifem IL-2 ist durch einen Pfeil gekennzeichnet.

Figur 2 zeigt schematisch die Konstruktion des Plasmids pRC2, ausgehend von pBR322.

Figur 3 zeigt schematisch die Konstruktion des Plasmids pRC23 mit einem $P_L$ Promotor.

Figur 4 beschreibt die Konstruktion des IL-2 Strukturgens, das Ser-IL-2 exprimiert.

Figur 5 zeigt schematisch die Konstruktion des Plasmids pRC201/IL2, das zur Konstruktion des Plasmids pRC233/IL2 (siehe Figur 6) verwendet wurde.

Figur 6 zeigt schematisch die Konstruktion eines Expressionsvektors mit einem $P_L$ Promotor-System, der zur Expression von reifem IL-2 verwendet wurde.

Das Verfahren der vorliegenden Erfindung stützt sich auf den überraschenden Befund, dass von einer Mikrobe exprimiertes IL-2 dazu neigt, mit der Membranfraktion der Wirtsmikrobe, in der Hauptsache mit der inneren Membran der Mikrobe (das ist diejenige Membran deren Lipid-Doppelschicht häufig mit hydrophoben Proteinen assoziert ist), zu assoziieren. Die Abtrennung der Membranen während des Aufreinigungsverfahrens für IL-2 aus transformierten Mikroorganismen führt demzufolge zu hohen Ausbeuten und einem Endprodukt von hoher Reinheit.

Obwohl verschiedene Verfahren zur Zellyse, z.B. enzymatische oder chemische Lyse. im Zusammenhang mit vorliegender Erfindung angewandt werden können, ist die Lyse durch Beschallung das bevorzugte Verfahren. Die inneren und äusseren Membranen werden dann von den restlichen Zellbestandteilen durch bekannte Verfahren, wie z.B. Zentrifugation, abgetrennt.

Nach der Abtrennung der Zellmembranen aus dem Zellysat, werden diese mit Extraktionslösungen, vorzugsweise Salz- und Detergenzlösungen, gewaschen um eine Lösung zu gewinnen, die wenigstens 50% IL-2 enthält. In einer bevorzugten Ausführungsform werden die Zellmembranen in 4 getrennten Schritten mit Salz- und Detergenzlösungen gewaschen. Der erste Schritt ist dadurch gekennzeichnet, dass man die Zellmembranen vorzugsweise mit einer Salzlösung, insbesondere mit 1M NaCl, wäscht. Im zweiten Schritt werden die Zellmembranen mit einer Detergenzlösung, vorzugsweise 1% Triton X-100, gewaschen. Im dritten Schritt werden die Zellmembranen mit einer weiteren Salzlösung, vorzugsweise 1.75-2 M Guanidin-HCl, gewaschen. Die abschliessende Waschung erfolgt mit einer weiteren Salzlösung, vorzugsweise etwa 4-7 M Guanidin-HCL. Die Waschlösung nach der 4. und letzten Waschung enthält dann ungefähr 50% IL-2.

Die letzte Waschlösung wird schliesslich vorzugsweise mit Hilfe der hochauflösenden Flüssigkeitschromatographie (HPLC) an Umkehrphasen chromatographisch gereinigt. Dieser HPLC-Reinigungsschritt führt zu aktivem, in fast 100% reiner oder homogener Form vorliegendem IL-2. Es ist auch denkbar, dass Antikörper-Affinitätschromatographie-Säulen, mit poly- oder monoclonalen Antikörpern gegen IL-2, anstelle von HPLC benutzt werden können. Andere chromatographische Reinigungsverfahren wie Farbstoff-Affinitätschromatographie (z.B. mit Procion Rot Agarose, wie in der Europäischen Patentanmeldung Nr. 83103582.9, veröffentlicht am 26. Oktober 1983 unter Publikations-Nr. 92163, beschrieben) oder Sephacryl S200 Säulen können ebenfalls benutzt werden. In einer weiteren bevorzugten Ausführungsform dieser Erfindung wird die Chromatographie mehrstufig durchgeführt, d.h. auf HPLC-Chromatographie folgt anschliessend Farbstoff-Affinitätschromatographie.

Das gemäss vorliegender Erfindung gereinigte IL-2 kann in gleicher Weise wie die anderen bekannten Substanzen mit immunmodulierender Aktivität verwendet werden, z.B. als Mittel zur Behandlung von immunsuppressiven Zuständen. Es kann in Form pharmazeutischer Präparate oral, durch Injektion oder lokal verabreicht werden. Dosis und Dosierfrequenz können entsprechend der Erfahrung bei der klinischen Applikation von bekannten immunmodulierenden Substanzen, üblicherweise 1-200 x $10^6$ Einheiten täglich, festgesetzt werden. Die aus dieser Erfindung resultierenden Arzneimittel enthalten besagtes IL-2 zusammen mit physiologisch verträglichem Trägermaterial. Alle üblichen Trägermaterialien können verwendet werden. Das Trägermaterial kann ein inertes, organisches oder anorganisches Material sein, welches sich zur enteralen, perkutanen oder parenteralen Darreichung eignet. Als Träger eignen sich Wasser, Gelatine, Gummi arabicum, Lactose, Maisstärke, Stearinsäure, Talk, vegetabile Oele, Polyalkylenglykole, insbesondere Polyäthylenglykole, natürliche Vaseline und dergleichen. Des weiteren können die pharmazeutischen Präparate weitere pharmazeutisch aktive Wirkstoffe enthalten. Zusätzliche Additive wie Aromatisierungsmittel, Ueberzugsmittel, Stabilisierungsmittel, Emulgiermittel, Puffer und dergleichen können mittels üblicher galenischer Verfahren zugefügt werden.

Die pharmazeutischen Präparate können in jeder Darreichungsform hergestellt werden, d.h. a) in fester Form, z.B. in Form von Tabletten, Kapseln, Pudern, Granulaten und dergleichen zur oralen Verabreichung: b) in flüssiger Form, z.B. in Form von Lösungen, Sirups, Suspensionen, Elixieren und dergleichen zur oralen Verabreichung; c) als Präparate zur parenteralen Verabreichung, z.B. als sterile Lösungen, Suspensionen oder Emulsionen: und d) als Präparate zur lokalen Anwendung in Form von Lösungen, Suspensionen, Salben, Crèmes, Gelées, mikronisierten Pudern, Aerosolen und dergleichen. Die pharmazeutischen Präparate können sterilisiert werden und/oder können Hilfsmittel wie z.B. Ueberzugsmittel, Stabilisierungsmittel, Netzmittel, Emulgiermittel, Salze zur Veränderung des osmotischen Druckes und/oder Puffer enthalten.

Als parenterale Verabreichungsformen kommen Infusions- oder Injektionslösungen, die intravenös oder intramuskulär injiziert werden können, in Frage.

Die Konstruktion von IL-2 Expressionsvektoren und Transformanden, die in der Lage sind, IL-2 zu exprimieren wird im Detail hiernach beschrieben.

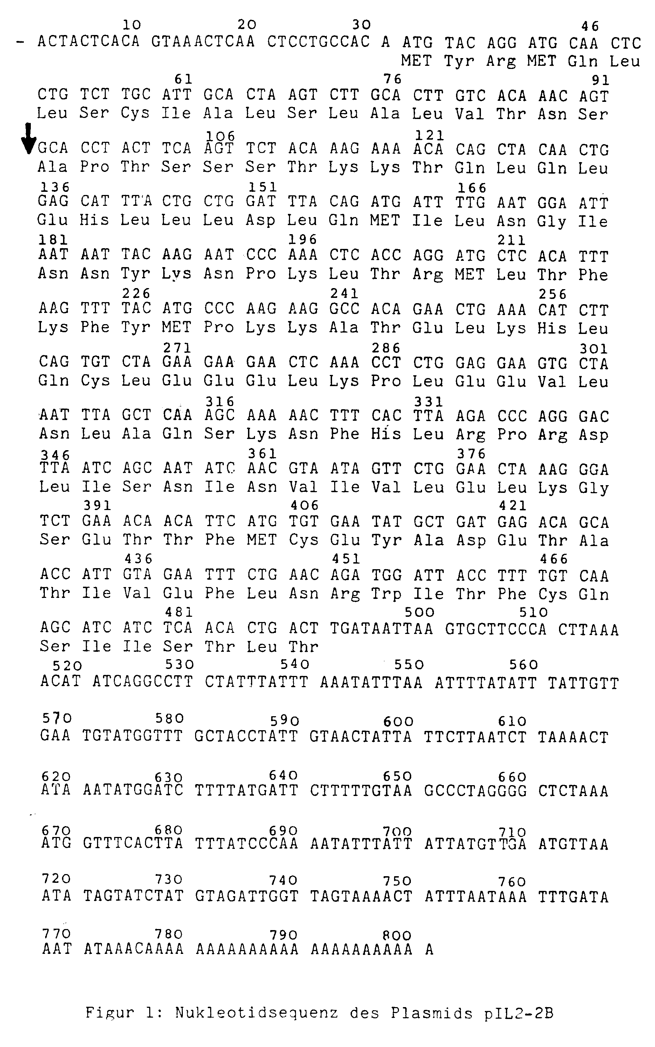

Die Aminosäure-Sequenz von reifem IL-2 Protein ist in Figur 1 gezeigt. Das Protein kann in seiner reifen Form mit Methionin als aminoterminaler Aminosäure exprimiert werden unter der Voraussetzung, dass das Initiationscodon am Genanfang ATG ist. Das Methionin wird u.U. von der Wirtszelle nach der IL-2 Expression abgespalten.

Die bei dieser Erfindung benutzten Expressionsvektoren sind pBR322 Derivate, die den $P_L$-Promotor, der aus Bakteriophagen-Lambda-DNA isoliert wurde, enthalten. $P_L$ ist der Promotor der Wahl, da er ein sehr starker Promotor ist, der wirksam vom Lambda-cl-Repressor kontrolliert wird, dessen Gen entweder auf dem Chromosom des Mikroorganismus, dem Vektor, der den $P_L$ Promotor enthält oder einem anderen kompatiblen Vektor lokalisiert ist. Das Gen, das für den Repressor codiert, trägt eine clts Mutation, die einen temperatursensitiven Repressor zur Folge hat. Solch ein Vektor, der bei dieser Erfindung benutzt werden kann und der die cl Mutation trägt, ist der Vektor pRK 248 clts, der in der Forschung bekannt und von Kahn et al., Methods in Enzymology, 68, 268 (1979), beschrieben wurde. Bei einer Temperatur von 30°C funktioniert der Repressor normal, jedoch bei Temperaturen von etwa 37°-42°C wird er inaktiviert. Der $P_L$ Promotor wird also bei 30°C reprimiert (abgedreht) und bei 37°-42°C derepremiert (angedreht). Diese Art der $P_L$ Promotor-Kontrolle erlaubt es, die Kultur bei etwa 30°C-36°C ohne Expression des gewünschten Genprodukts wachsen zulassen, um dann zu einem optimalen Zeitpunkt die Temperatur auf etwa 42°C zu erhöhen, bei der das gewünschte reife Human-IL-2 Produkt exprimiert wird.

Der bei dieser Erfindung bevorzugt benutzte Vektor besitzt eine zusätzliche EcoRI Schnittstelle distal zur SD Sequenz (stromabwärts in 3' Richtung). Die folgende Beschreibung dient zur Illustration der Konstruktion eines bevorzugten Vektors, des Vektors pRC23, in den das IL-2 Gen eingeführt wird. Andere Vektoren können jedoch auch benutzt werden.

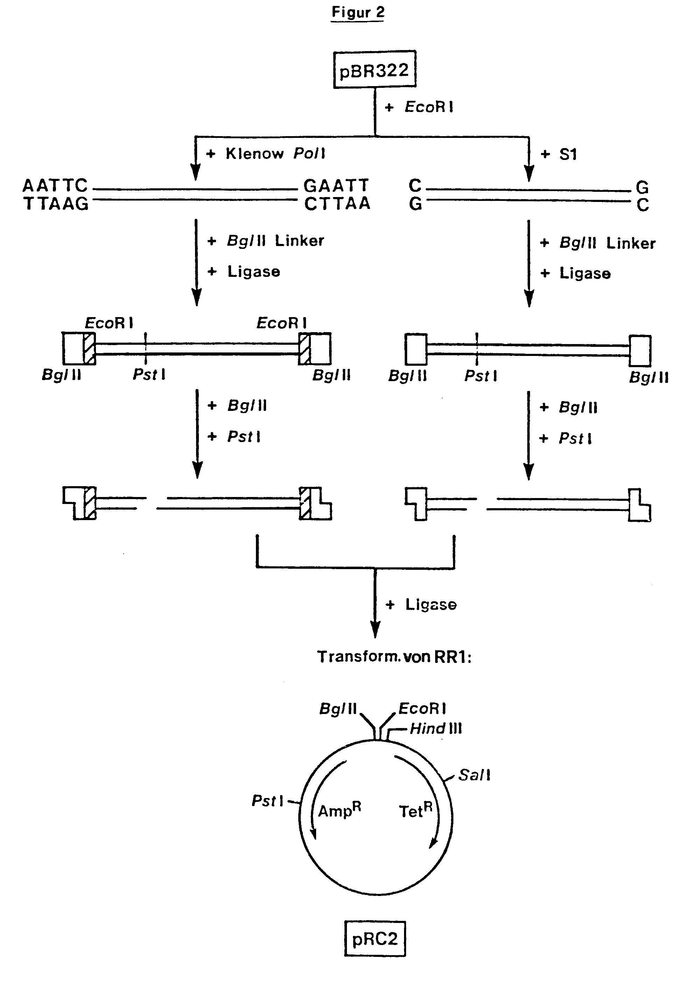

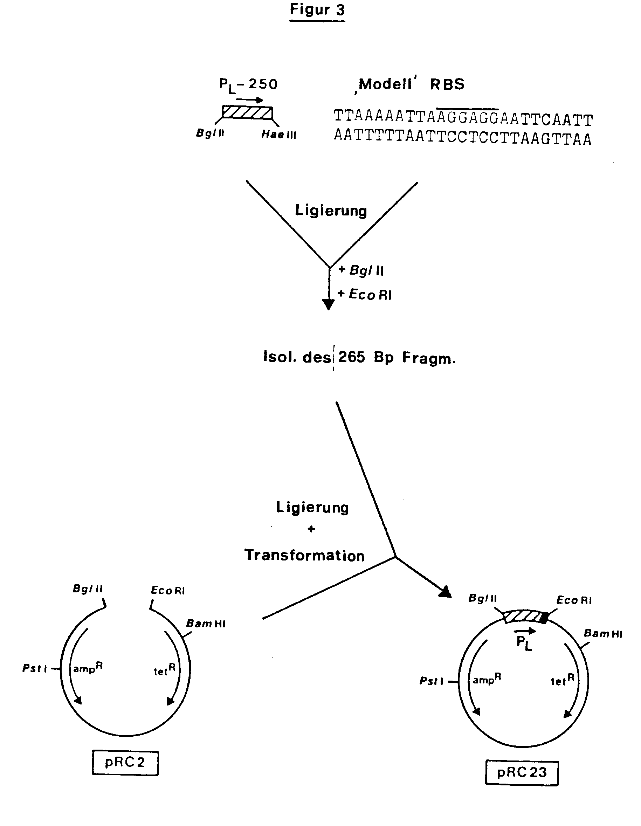

Gemäss dem in den Figuren 2 und 3 beschriebenen Verfahren wurden 20 Mikrogramm pBR322 DNA mit EcoRI verdaut und anschliessend in zwei verschiedenen Reaktionen eingesetzt: 1. zur Behandlung mit S1 Nuklease, um die 5'- überhängenden Enden zu entfernen, und 2. zur Behandlung mit DNA Polymerase I Klenow Fragment, um die kohäsiven Enden aufzufüllen. Beide Reaktionen wurden durch Phenol- und anschliessende Aethanolextraktion gestoppt. Die DNA aus jedem der Reaktionsansätze wurde mit synthetischen Bgl II Verbindungssequenzen kombiniert, mit Bgl II und Pst I verdaut und danach an 1% Agarose elektrophoretisch aufgetrennt. Die 3600 BP (Basenpaare) und 760 BP-enthaltenden Fragmente aus beiden Reaktionsansätzen wurden aus dem Gel isoliert. Zur Konstruktion von pRC2, wurde das 3600 BP-enthaltende Fragment aus der Klenow Reaktion mit dem 760 BP-enthaltenden Fragment, der S1 Reaktion kombiniert. Der E. coli Stamm RR1 wurde mit dieser DNA-Kombination transformiert und Transformanden wurden in einem Medium mit 50 $\mu$g/ml Ampicillin selektioniert. Transformanden mit der erwarteten Plasmid-Kombination, d.h. einem Plasmid mit einer Bgl II-Schnittstelle in unmittelbarer Nähe der EcoRI-Schnittstelle, wurden durch Restriktionsanalyse der isolierten Plasmid-DNAs identifiziert. Das Plasmid pRC 23 wurde durch die Kombination eines synthetischen Oligonukleotids, welches für eine "consensus" RBS (vom Computer entwickelte Ribosomen-Bindungsstelle) codiert [Scherer et al. Nucleic Acids Research, 8, 3895 (1980)], mit einem 250 BP-enthaltenden Bgl II-Hae III Fragment, welches den Lambda $P_L$ Promotor enthält, und dem Plasmid pRC2 konstruiert.

Um das 250 BP-enthaltende DNA-Fragment zu isolieren, welches den Lambda $P_L$ Promotor enthält, wurde 1 Mikrogramm 450 BP-enthaltendes Bgl II-HpaI DNA Fragment (von BP # 35260 bis 35710 der Lambda Phagen DNA-Sequenz) mit Hae III verdaut und die resultierenden Produkte wurden mit Hilfe präparativer Gelelektrophorese an 5% Polyacrylamid isoliert. Ungefähr 200 ng des 250 Bp-enthaltenden Bgl II-Hae III Fragments wurden mit je 60 pM der in Figur 3 gezeigten synthetischen Oligonukleotide kombiniert, welche für den grössten Teil einer "consensus" RBS-Sequenz codieren, deren Sequenz, wie von Scherer et al. beschrieben, mittels Computer-Analyse entwickelt wurde. Die resultierenden Moleküle wurden dann mit Bgl II und EcoRI behandelt (um Oligomere auszuschliessen), gelelektrophoretisch gereinigt und mit pRC2 DNA, die ebenfalls mit Bgl II und EcoRI behandelt wurde, kombiniert. Die Transformation von E. coli RR1 (pRK 248 clts) mit dieser DNA-Kombination wurde nach Standardverfahren durchgeführt und die Transformanden wurden in einem Medium mit 50 $\mu$g/ml Ampicillin bei 30°C selektioniert. 50 Transformanden wurden erhalten, von denen 8 für die weitere Analyse ausgewählt wurden. Die Plasmid DNAs dieser 8 Transformanden wurde isoliert und mittels Hinc II-Spaltung analysiert. 6 dieser 8 Plasmid DNAs zeigten das erwartete Restriktionsmuster und auch die Nukleotidsequenz-Analyse nach Maxam-Gilbert von einer dieser 6 bestätigte die erwartete Konstruktion (bezeichnet als pRC 23).

Konstruktion eines Expressionsvektors, der für reifes Human-IL-2 codierende DNA enthält

(1) Isolierung von Human-IL-2 codierender mRNA

mRNA wurde aus H33HJ-JAI Zellen (ATCC Nr. CRL-8163, hinterlegt am 26. August 1982), einem Klon der Jurkat Zellinie FHCRC, nach Induktion mit PHA und PMA isoliert. 12000 ml #33HJ-JAI-Klon Zellkulturen ($10^6$ Zellen/ml) wurden in RPMI 1640 Gewebekultur-Medium, ergänzt mit 10% fötalem Rinderserum, 50 E/ml Penicillin, 50 $\mu$g/ml Streptomycin, 50 $\mu$g/ml Gentamycin und 300 $\mu$g/ml frischem L-Glutamin, wachsen gelassen. Diese Zellen wurden durch Zentrifugation gesammelt und in 6 l des zuvor ausführlich beschriebenen Mediums, ohne Serum, jedoch ergänzt mit 1% PHA und 10 $\mu$g/ml PMA, resuspendiert. Jeweils 6 l resuspendierte Zellen wurden auf sterile Glaswalzenflaschen verteilt und auf ein Walzwerk gelegt (10 U/min. bei 37°C).

8 Stunden später, wurden die Zellen durch Zentrifugation geerntet und die mRNA wurde durch ein Phenol-Chloroform-Standardverfahren extrahiert. Danach wurde die Aethanol-gefällte RNA durch Hochgeschwindigkeitszentrifugation sedimentiert und in einer 0.5 M Salzlösung aufgenommen. Oligo-(dT)-Cellulose-Chromatographie wurde benutzt um die mRNA mit poly(A)-Ende von der Gesamt-RNA abzutrennen. Die Aethanol-gefällte mRNA wurde in Wasser in einer Konzentration von 500 $\mu$g/ml aufgenommen. 30 ng RNA wurden dann in Xenopus Oozyten mikroinjiziert. Nach 24 Stunden Inkubation in steriler Barth's Lösung, wurden 4 Eier in sterile 1.5 ml Eppendorf-Hütchen überführt und mit 500-1500 $\mu$l frischer, steriler Barth's Lösung gefüttert. Nach 48 Stunden wurden 200 $\mu$l Inkubationsmedium geerntet und mit einem Standard CTLL-Zell-$^3$H-Tdr-Einbautest (Gillis et al., J. Immunol. 120, 2027 [1978]) auf IL-2 Aktivität getestet. mRNA Proben, die nach der Translation im Oozytentest eine deutliche IL-2 Aktivität zeigten, wurden gesammelt, nach ihrer Grösse durch Sucrose-Dichtegradienten-Zentrifugation aufgetrennt und nach Aethanol-Fällung zur Herstellung einer cDNA-Bibliothek verwendet.

(2) cDNA-Synthese

3,5 $\mu$g gereinigte mRNA (mit einem Sedimentations-Koeffizient von 10S im Sucrosegradienten) wurden zur Herstellung doppelsträngiger komplementaerer DNA (ds cDNA) nach der folgenden Methode (Gubler and Hoffmann, Gene 25, 263-269 [1983]) verwendet.

(a) Erststrang cDNA Synthese:

Die mRNA wurde in 17,5 $\mu$l Tris-HCl, pH 8,3, 10 mM MgCl$_2$, 10 mM DTT, 4 mM Na-Pyrophosphat, 1,25 mM dATP, 1,25 mM dGTP, 1,25 mMdTTP, 0,5 mM dCTP, 100 $\mu$g/ml Oligo (dT)$_{12-18}$, und 10 Ci $^{32}$P-dCTP (Amersham 3000 Ci/mMol) inkubiert. Nach 5 Minuten Inkubation bei 43°C wurden 3000 Einheiten AMV reverse Transcriptase/ml zur Inkubationsmischung gegeben und diese für weitere 30 Minuten bei 43°C inkubiert. Die Reaktion wurde durch Zugabe von EDTA zu 20 mM gestoppt, das cDNA-mRNA Hybrid mit Phenol-Kresol extrahiert und durch Aethanol-Fällung konzentriert. Die Ausbeute der Erststrang Synthese betrug 58,6 ng (1,7%) gemessen durch TCA-unlösliche Radioaktivität.

(b) Zweitstrang cDNA Synthese:

Das cDNA-mRNA Hybrid wurde in 5.8 $\mu$l H$_2$O aufgenommen. Zu dieser Lösung wurden 7.7 $\mu$l Zweitstrang-Synthesemix gegeben um eine Lösung mit 20 mM Tris-HCl, pH 7,5, 5 mM MgCl$_2$, 10 mM (NH$_4$)$_2$SO$_4$, 100 mM KCl, 0,15 mM beta-NAD, 50 $\mu$g/ml BSA, 40 mM dNTPs, 8,5 Einheiten/ml E. coli RNase H (Bethesda Research Labs), 230 Einheiten/ml DNA Polymerase I, 10 Einheiten/ml E. coli DNA Ligase zu erhalten. Die Mischung wurde zuerst für 60 Minuten bei 12°C und dann für 60 Minuten bei 22°C inkubiert. Die Reaktion wurde durch Zugabe von EDTA zu 20 mM gestoppt und die ds cDNA mit Phenol-Kresol extrahiert. Eine mit Sephadex G-50 fein gefüllte, mit 10 mM TEAB äquilibrierte Säule wurde benutzt, um die ds cDNA von den freien Nukleotiden abzutrennen. Die Ausbeute dieser Reaktion betrug 107 ng ds cDNA (91%).

(c) Verknüpfung und Transformation

64 ng ds cDNA wurden mit Deoxyguanosin (dG)-Resten nach Standardmethoden verlängert. Der Vektor, pBR322, wurde zuerst mit EcoRV verdaut und anschliessend mit Deoxycytidin (dC)-Resten verlängert. 100 ng verlängerte pBR 322 DNA und 1,25 ng verlängerte cDNA-Fragmente (Verhältnis 80:1) wurden in 250 $\mu$l 0,01 M Tris, pH 7,5, 1 mM EDTA, 0,15 M NaCl für 90 Minuten bei 58°C verbunden und zur Transformation mit kompetenten E. coli RR1 Zellen verwendet. Die transformierten Zellen wurden auf LB Platten mit 100 $\mu$g/ml Ampicillin ausplattiert und für 12 Stunden bei 37°C inkubiert. Es wurden 3200 Kolonien für die IL-2 cDNA-Bihliothek erhalten.

(3) Sichten der cDNA-Bibliothek nach IL-2 Gensequenzen

Zum Auffinden von cDNA-Kopien mit vollständigen IL-2 Gensequenzen wurde eine synthetische Deoxynukleotidsonde benutzt, die den von Taniguchi et al. (Nature 302, 305--310 [1983]) veröffentlichten Nukleotiden 45-65 der Human-IL-2 cDNA-Sequenz entspricht. Diese Sonde [ACAATGTACAGGATGCAACTC] wurde nach der Festphasen-Phosphordiestermethode chemisch synthetisiert, mit HPLC gereinigt und mit $^{32}$P-ATP (ICN Pharmaceuticals, 7000 Ci/mM) und Polynukleotidkinase markiert. Kolonien der cDNA-Bibliothek wurden nach der Methode von Grunstein und Hogness (Methods in Enzymology 68, 379 [1979]) auf Nitrocellulose-Filter übertragen. Nach Hybridisierung bei 30°C für 16 Stunden, wurden die Filter in 4 x SSC Puffer bei 45°C für 45 Minuten gewaschen, getrocknet und für die Autoradiographie verwendet. Eine einzige positive Kolonie wurde erhalten, welche die vollständige IL-2 Gensequenz enthielt. Von dieser Kolonie, pIL-2-2B bezeichnet, wurde Plasmid DNA hergestellt und für die Nukleotidsequenz-Analyse und die Expression in E. coli verwendet. Die Nukleotidsequenz war bis auf zwei Veränderungen mit der von Taniguchi et al. veröffentlichten Sequenz identisch. Diese Veränderungen sind: ein Insert, welches bei Nukleotid 17 der Taniguchi Sequenz beginnt und ein Austausch von G in Position 503.

(4) Herstellung eines E. coli Plasmidvektors, der für Ser-IL-2 codierende DNA enthält

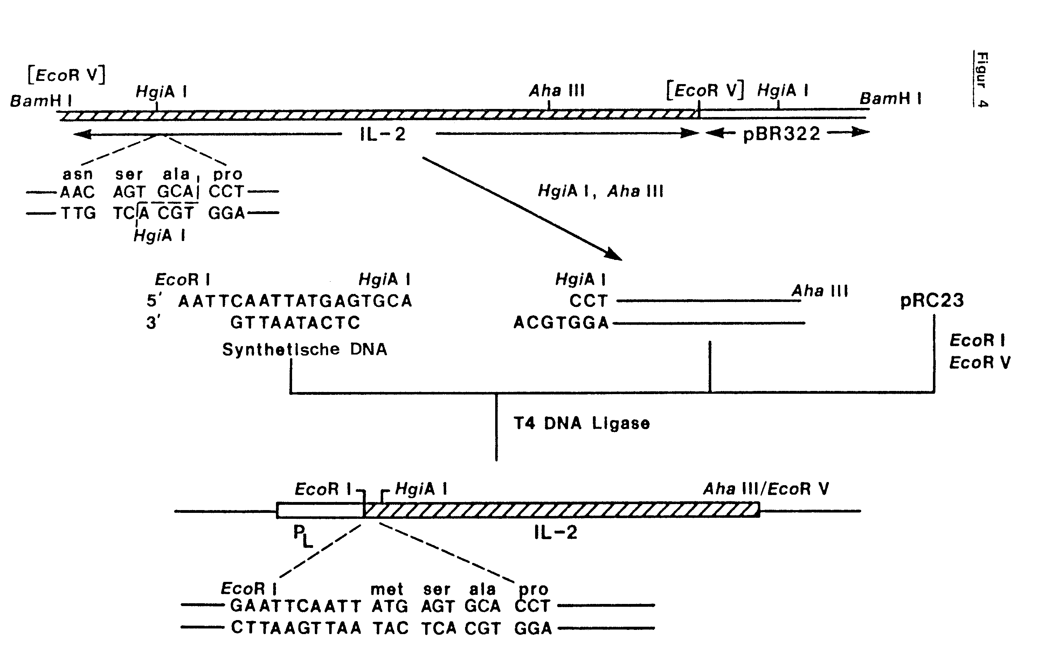

Ein E. coli Expressionsvektor, der Ser-IL-2 codierende DNA enthält, wurde durch Kombination dreier Segmente hergestellt (siehe Figur 4):(1) dem Vektor pRC23 mit dem Lambda P$_L$ Promotor, (2) einem synthetischen Verbindungsstück und (3) einem aus IL-2 cDNA isolierten HgiAI--Aha III Fragment. pRC23 Vektor DNA (50 ng) wurde mit EcoRI und EcoRV verdaut, mit Phenol extrahiert und dann mit Aethanol präzipitiert. Das synthetische Adapterstück wurde durch Hybridisierung zweier komplementärer synthetischer Deoxynukleotid-Sequenzen, A und B hergestellt:

(A)     5'AATTCAATTATGAGTGCA 3'

(B)     3' GTTAATACTC 5'

Dieses doppelsträngige Verbindungsstück kann an seinem 5'-Ende mit einer EcoRI-Schnittstelle und an seinem 3' Ende mit einer HgiAI-Schnittstelle verbunden werden. Das 1 Kb grosse IL-2 cDNA-Insert wurde aus pIL-2-2B DNA nach Behandlung mit dem Restriktionsenzym Bam HI durch Gelreinigung isoliert. Das so erhaltene DNA-Fragment wurde mit HgiAI und Aha III nachbehandelt, mit Phenol extrahiert und mit Aethanol präzipitiert. HgiAI schneidet zwischen dem Alanin und dem Prolin Codon am Anfang der für reifes IL-2 codierenden Sequenz. Bei der Klonierung zur Expression des entsprechenden IL-2 Gens wird die dem P$_L$ Promotor benachbarte EcoRI-Schnittstelle des Vektors pRC23 benutzt. Das synthetische Verbindungsstück kann diese EcoRI-Schnittstelle mit der HgiAI Schnittstelle am Anfang des IL-2 cDNA-Fragments verbinden. Durch die Verknüpfung des Verbindungsstückes mit der cDNA werden gleichzeitig das für den Translationsstart notwendige Methionin-Codon und die Codons für Serin und Alanin, die ersten Aminosäuren des reifen IL-2 Proteins, erzeugt. Die cDNA wird mit dem stumpfen Aha III Ende über das stumpfe EcoRV Ende mit der pRC23 Vektor DNA verbunden.

Die Kombination der drei DNA-Segmente wurde in 10 µl Reaktionsmischung, enthaltend 65 mM Tris, pH 7.6. 10 mM MgCl$_2$, 0,5 mM ATP, 15 mM beta-Mercaptoäthanol und je 0,05 pM DNA des Vektors, des synthetischen Verbindungsstückes und des cDNA-Inserts, durchgeführt. Das Kombinationsgemisch wurde zuerst für 5 Minuten auf 65°C erhitzt, dann auf 4°C abgekühlt und nach Zugabe von 200 Einheiten T4 DNA Ligase für 16 Stunden bei 4°C inkubiert. Die T4 DNA Ligase wurde durch Erhitzen der Reaktionsmischung auf 65°C für 5 Minuten inaktiviert. Anschliessend wurden die verbundenen DNA-Fragmente mit EcoRV behandelt, um nichtverdaute Vektor-DNA zu beseitigen. Die Mischung wurde dann zur Transformation mit E. coli RR1 (pRK 248 clts) verwendet. Diese Zellen wurden bei 30°C für 15 Stunden wachsen gelassen.

Die Kolonien wurden, wie zuvor beschrieben, auf Nitrocellulosefilter übertragen, getrocknet und mit der $^{32}$P-markierten synthetischen Deoxynukleotidsequenz A hybridisiert. Eine positive Kolonie, pRC 23/IL-2 # 4-1 bezeichnet, wurde für die weitere Analyse ausgewählt. Dieser Klon synthetisierte mehr als 200 000 Einheiten/ml IL-2, nachdem der P$_L$ Promotor durch die Temperaturerhöhung auf 42°C induziert worden war. Die IL-2 Aktivität wurde mittels biologischem Test auf CTLL Zellen, eine Indikator-Zellinie für IL-2 (beschrieben von Gillis et al. J. Immunol. 120, 2027 [1978]), nachgewiesen.

(5) Herstellung eines E. coli Expressionsvektors, der reifes IL-2 synthetisiert (mit Alanin am aminoterminalen Ende).

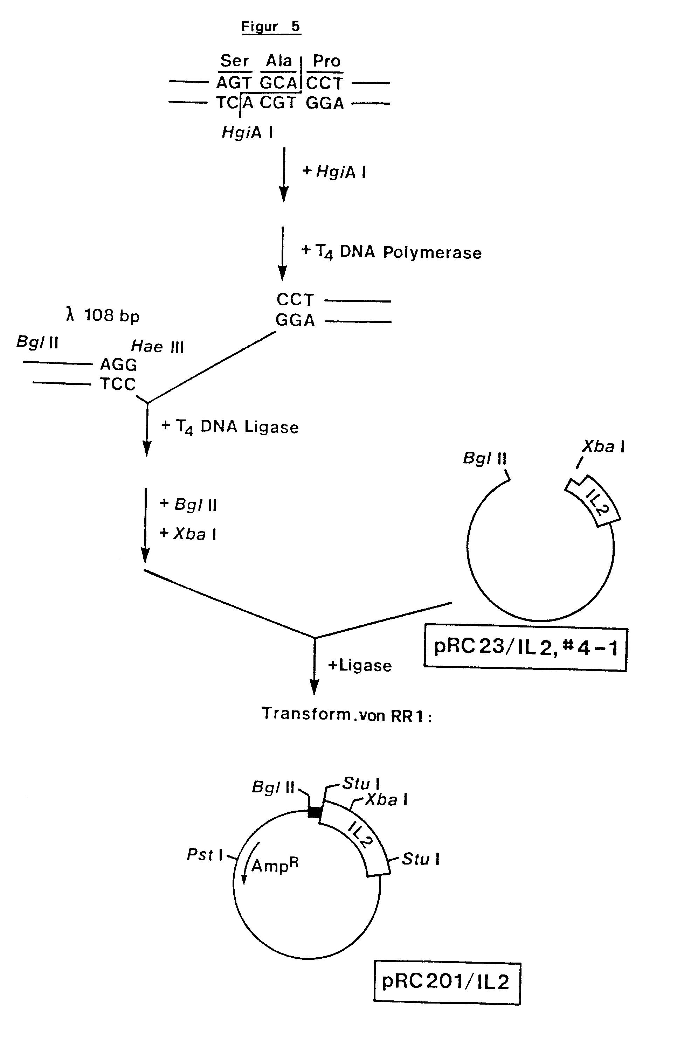

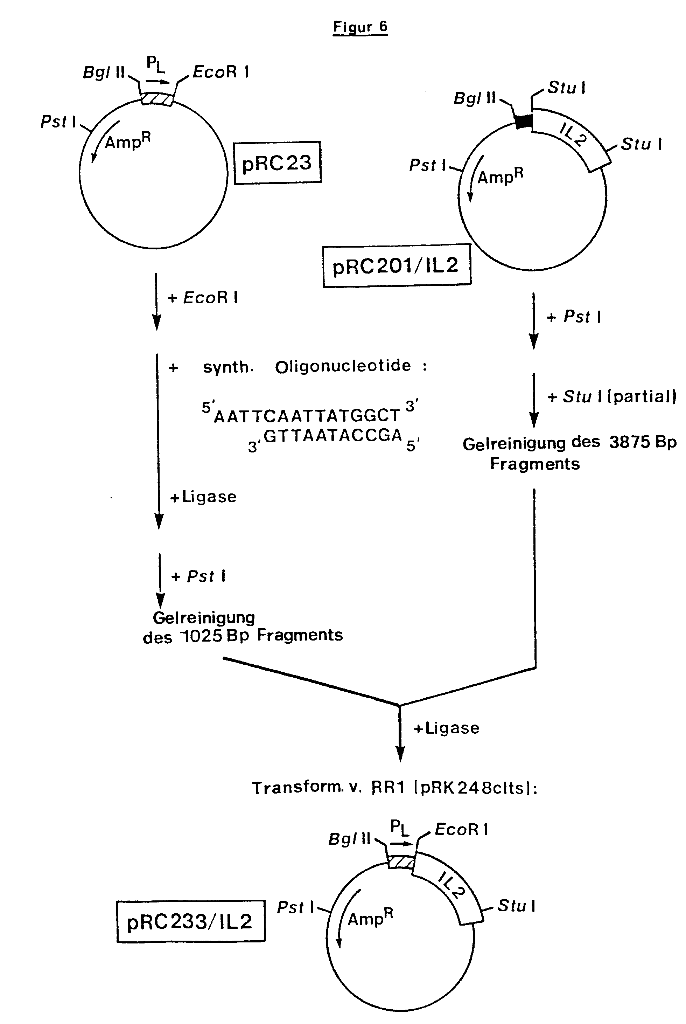

Zur Expression von reifem IL-2 in E. coli, wurde der folgende Weg gewählt. Wie in Figur 5 gezeigt, befindet sich an der Ser-Ala Peptidbindung, die gleichzeitig die Schnittstelle zum Entfernen des Signalpeptids darstellt (Robb et al., PNAS, 80, 5990-5994 (1983)), eine HgiAI-Schnittstelle. Nach Behandlung mit HgiAI wurde das kohäsive Ende durch Behandlung mit T4 DNA Polymerase in ein stumpfes Ende umgewandelt. Das resultierende Fragment wurde dann über sein stumpfes Ende mit einem aus dem Phagen Lambda isolierten 108 BP BglIII-Hae III-Fragment verbunden. danach mit Bgl II und Xba I behandelt und zwischen die Restriktionsschnittstellen Bgl II und Xba I des Vektors pRC23/IL-2, # 4-1, gehängt. Dieser Klonierungszwischenschritt wurde gemacht um sicher zu gehen, dass die T4-DNA Polymerase-Behandlung wie erwartet funktioniert hat, was durch die Schaffung einer neuen Stu I-Schnittstelle durch Verbindung des Hae III Endes mit dem stumpfen HgiAI-Ende bestätigt wurde. Nachbehandlung mit Stu I stellt das mit dem Prolin-Codon CCT beginnende stumpfe Ende an dieser Stelle wieder her. Diese Plasmid-Zwischenkonstruktion wurde pRC 201/IL-2 bezeichnet. Es wurden 2 synthetische Deoxyoligonucleotide geschaffen, die ein ATG-Translations-Startcodon enthalten, das Codon für die Aminosäure Alanin wiederherstellen und ein EcoRI Ende erzeugen. Diese Oligomere wurden über die EcoRI Enden mit dem Expressionsvektor pRC23 verbunden. Das so erhaltene Produkt wurde mit Pst I nachbehandelt, so dass ein 1025 BP-enthaltendes Fragment entstand, welches mittels Gelelektrophorese isoliert wurde. Das 1025 BP-enthaltende Fragment (mit einem Pst I und einem stumpfen Ende) wurde zwischen die Pst I - und die neu geschaffene Stu I-Restriktionsstellen des Plasmids pRC201/IL-2 eingefügt. Zur Identifizierung von Transformanden mit gewünschter Konstruktion wurden die Plasmid DNAs isoliert und durch Restriktionsenzymanalysen überprüft. Die bestätigte Plasmid-Konstruktion wurde pRC233/IL-2 bezeichnet (siehe Figur 6).

Bevorzugte Wirtsorganismen zur Transformation mit einem Vektor, der das IL-2 Gen entält, sind erfindungsgemäss Bakterien, wie z.B. E. coli; Bacillaceae, wie z.B. Bacillus subtilis und dergleichen. Hefen bilden eine weitere Gruppe bevorzugter Mikroorganismen zur Transformation.

Ein besonders bevorzugter Mikroorganismus, der als Rezipient bei den Transformationen verwendet wird, ist E. coli K-12 Stamm 294, wie in der britischen Patentanmeldung Nr. 20 55 382 A beschrieben. Dieser Stamm wurde bei der American Type Culture Collection am 28. Oktober 1978 unter der ATCC Nr. 31 446 deponiert. Ausserdem können auch andere E. coli Stämme, wie z.B. E. coli RR1, ATCC Accession Nr. 31 343, oder andere Mikroorganismen, von denen die meisten an einem Depositorium, wie der American Type Culture Collection, hinterlegt worden sind und allgemein zugänglich sind, als Wirtsorganismen benutzt werden. Bei der Umsetzung dieser Erfindung in die Praxis werden Uebernachtkulturen der transformierten E. coli Zellen in LB-Medium bei 30°C wachsen gelassen. Ein Liter der Uebernachtkulturen wird vorzugsweise mit M-9 Minimalmedium, ergänzt mit Casaminosäuren, auf 10 Liter verdünnt. Nach dem Eintritt in die logarithmische Wachstumsphase wird die Wachstumstemperatur der Kultur zur Induktion der IL-2 Produktion von 30°C auf 42°C erhöht. Nach 2-3-stündiger Inkubation bei 42°C werden die Bakterien mittels Zentrifugation gesammelt. Die gesamte Arbeit wurde unter Beachtung der Richtlinien des National Institute of Health durchgeführt. Die Reinigungsschritte sind im Detail in den folgenden Beispielen beschrieben.

## Beispiel 1

1 g transformierter E. coli Zellen, die IL-2 (mit Serin am aminoterminalen Ende des Proteins) herstellen, wurden in 30 mM Tris-HCl, pH 8,0, 5 mM EDTA, 1 mM Phenylmethylsulfonylfluorid resuspendiert und durch Beschallen lysiert. Das Lysat wurde bei 14000 x g 15 Minuten zentrifugiert. Die Membranbestandteile des zentrifugierten Lysats wurden von den anderen Lysat-Bestandteilen abgetrennt. Die Membranbestandteile wurden dann 4 Wasch- oder IL-2-Extraktionsschritten unterworfen, deren Ergebnisse in Tabelle 1 zusammengefasst sind. Für jede Waschung wurden 5 ml Waschlösung pro Gramm Zellen verwendet. Wie in Tabelle 1 im Detail zusammengefasst, wurde der Hauptteil der IL-2-Aktivität bei der letzten Waschung extrahiert. Diese Waschlösung die den Hauptteil der IL-2-Aktivität und wenigstens 50% reines IL-2, gemessen mit Hilfe von SDS-PAGE, enthielt, wurde direkt der Umkehrphasen-HPLC (RP-C8) unterworfen. Die Ausbeute betrug 40-60% fast 100% reines IL-2 (mit Serin am aminoterminalen Ende des Proteins). Die Ausbeute an biologischer IL-2-Aktivität lag bei $1 \times 10^8$ Einheiten/mg.

Tabelle 1

| Extraktion von IL-2 aus E. coli Membranen | | | |
|---|---|---|---|
| Extraktion | Gesamt-Protein (mg) | Gesamt IL-2 Aktivität (Einheiten) | Spezifische Aktivität (E/mg) |
| Waschung mit 1 M NaCl | 0,4 mg | $0.03 \times 10^6$ | $0.1 \times 10^6$ |
| Waschung mit 1% Triton | 2 mg | $0,75 \times 10^6$ | $0,4 \times 10^6$ |
| Waschung mit 1.75 M Guanidin-HCl | 1 mg | $1,5 \times 10^6$ | $1,5 \times 10^6$ |
| Waschung mit 7 M Guanidin-HCl | 4,7 mg | $250 \times 10^6$ (99%) $\overline{252,3 \times 10^6}$ | $53 \times 10^6$ |

Beispiel 2

Dieses Beispiel dient zur Darstellung der Neigung von IL-2, sich an die Zellmembranbestandteile der transformierten E. coli Wirtszellen zu assoziieren.

Um dies zu zeigen, wurden 1 g transformierter E. coli Zellen, die IL-2 mit Serin am aminoterminalen Ende herstellen, in 10 ml 20%-prozentiger Sucrose, 30 mM Tris-HCl, pH 8,0 aufgenommen und mit Lysozym-EDTA lysiert um die im periplasmatischen Raum lokalisierten Proteine abzutrennen. Der Rest des Lysats wurde anschliessend beschallt. Die Membranen (innere und äussere Membran) wurden durch Sucrosegradient-Zentrifugation von den anderen zellulären Komponenten ab- und in innere und äussere Membranen aufgetrennt. Tabelle 2 zeigt, dass die Hauptmenge der biologischen IL-2-Aktivität bei der inneren Zellmembran zu finden ist.

Tabelle 2

| Lokalisierung von IL-2 in E. coli Zellen | | | |
|---|---|---|---|
| Fraktionen | Gesamt-Protein (mg) | Gesamt IL-2 Aktivität (Einheiten) | Ausbeute an IL-2 [%] |
| Lösliche Fraktion | 37 mg | $2 \times 10^6$ | 0,3 |
| Periplasmische Fraktion | 2 mg | $10 \times 10^6$ | 1,5 |
| Aeussere Membran | 6 mg | $8 \times 10^6$ | 1,2 |
| Innere Membran | 15 mg | $6,6 \times 10^8$ | 97 |

Beispiel 3

1 g transformierter E. coli Zellen, die reifes IL-2 herstellen, wurden in 30 mM Tris-HCl, pH 8, 5 mM EDTA, 1 mM Phenylmethylsulfonylfluorid resuspendiert und durch Beschallen lysiert. Das Lysat wurde bei 14000 x g 15 Minuten zentrifugiert. Die Membranbestandteile des zentrifugierten Lysats wurden von den anderen Lysatbestandteilen abgetrennt. Die Membranbestandteile wurden dann 4 Wasch- oder IL-2-Extraktionsschritten unterworfen, deren Ergebnisse in Tabelle 3 zusammengefasst sind. Für jede Waschung wurden 5 ml Waschlösung pro Gramm Zellen verwendet. Wie in Tabelle 3 im Detail zusammengefasst, wurde der Hauptteil der IL-2 Aktivität bei der letzten Waschung extrahiert. Diese Waschlösung, die den Hauptteil der IL-2 Aktivität und wenigstens 50% reines IL-2, gemessen mit Hilfe von SDS-PAGE, enthielt, wurde direkt der Umkehrphasen-HPLC (RP-C8) unterworfen. Die Ausbeute betrug ungefähr 60% fast 100% reines IL-2 Protein. Die Ausbeute an biologischer IL-2-Aktivität lag bei $4 \times 10^8$ Einheiten/mg.

Tabelle 3

| Extraktion von reifem IL-2 aus E. coli Membranen | | | |
|---|---|---|---|
| Extraktion | Gesamt-Protein (mg) | Gesamt IL-2 Aktivität (Einheiten) | Spezifische Aktivität (E/mg) |
| Waschung mit 1 M NaCl | 0.5 | $0,3 \times 10^6$ | $0,6 \times 10^6$ |
| Waschung mit 1% Triton | 3.3 | $5,5 \times 10^6$ | $1,7 \times 10^6$ |
| Waschung mit 1,75 M Guanidin-HCl | 2,5 | $10,0 \times 10^6$ | $4,0 \times 10^6$ |
| Waschung mit 7 M Guanidin-HCl | 10,1 | $779 \times 10^6$ (98%) $\overline{794,8 \times 10^6}$ | $77,1 \times 10^6$ |

Beispiel 4

Das folgende Beispiel beschreibt die Reinigung von reifem IL-2 aus einer Paste transformierter E. coli Zellen durch Procion-Rot Agarose-Chromatographie und HPLC.

Zellmembranextraktion

Gefrorene E. coli Zellen (enthaltend ein für Human IL-2 codierendes Plasmid) werden aufgetaut und 1 g dieser Zellen wird in 5 ml Puffer A (0,03 M Tris-HCL, pH 8,0. 0,005 M EDTA) aufgenommen. Die resultierende Zellsuspension wird dann 15 Minuten gerührt und danach werden die Zellen durch Zentrifugation in einem Sorvall SS-34 Rotor (10000 U/min. während 10 Minuten) gesammelt. Der Ueberstand wird verworfen und die Zellen werden in 5 ml Puffer A resuspendiert. Die resuspendierten Zellen werden mit einem Ultraschallgerät aufgebrochen (6 x 30 Sekunden). Die aufgebrochenen Zellen werden abzentrifugiert (10000 U/min. während 10 Minuten) und der Ueberstand mit den löslichen Proteinen wird verworfen. Der Rückstand wird einmal mit 5 ml Puffer A gewaschen und abzentrifugiert (10000 U/min. während 10 Minuten). Der Rückstand, welcher der Membranfraktion entspricht, wird in Puffer B (1 M NaCl, 0,03 M Tris-HCl, pH 8,0, 0,005 M EDTA) mit einem Gewebehomogenisator aufgelöst und 10 Minuten gerührt. Die Membranfraktion wird durch Zentrifugation in einem Sorvall SS-34 Rotor (15000 U/min. während 10 Minuten) abzentrifugiert. Der Rückstand wird in 5 ml Puffer C (1% Triton X-100, 0,03 M Tris-HCl, pH 8,0) gelöst, homogenisiert, gerührt und erneut abzentrifugiert (15000 U/min. während 10 Minuten). Der Rückstand wird in 5 ml 1,75 M Guanidin-HCl gelöst. homogenisiert, gerührt und abzentrifugiert (15000 U/min. während 10 Minuten). Der resultierende Rückstand wird einmal mit 5 ml Puffer A gewaschen und abzentrifugiert. Die Membranfraktion (Rückstand) wird mit 5 ml 7 M Guanidin-HCl extrahiert. Nach der Zentrifugation wird der IL-2 enthaltende Extrakt aufbewahrt und der Rückstand ein zweites Mal mit 5 ml 7 M Guanidin-HCl extrahiert. Dieser Extrakt wird ebenfalls aufbewahrt.

Procion-Rot Agarose-Chromatographie

Die Säule wird bei Raumtemperatur gefüllt, mit zwei Säulenvolumen 7 M Guanidin-HCl gewaschen und dann bei 4°C mit Aequilibrierungspuffer (0,01 M Tris-HCl, pH 7,9, 0,035 M NaCl) äquilibriert. Mindestens 1 ml Procion Rot Agarose sollte pro 100 $\mu$g erwartetes IL-2 Protein verwendet werden. Die Durchflussrate beträgt 2 Bettvolumina pro Stunde. Der IL-2 enthaltende 7 M Guanidin-HCl Extrakt wird 40-fach mit Aequilibrierungspuffer verdünnt und das Präzipitat durch Zentrifugation entfernt. Mit dem resultierenden Ueberstand wird die Säule beladen. Die Säule wird mit zwei Säulenvolumina Aequilibrierungspuffer gewaschen. IL-2 erscheint in einem Gipfel nach 1,5-2 Volumina Elutionspuffer (0,01 M Tris-HCl, pH 7,9, 1,035 M NaCl). Nach der Elution des IL-2, wird die Säule durch Waschen mit 6 M Guanidin-HCl von Fremdmaterial gesäubert.

HPLC (C-18)-Chromatographie

Der pH des Procion-Rot-Eluats wird durch langsame Zugabe von 1.0 M Essigsäure auf 7 eingestellt. Proben von jeweils 0.1 ml werden zur Bestimmung der Aktivität vor und nach der pH-Einstellung entnommen. Das neutralisierte Eluat (60 ml) wird mit einer Durchflussrate von 1 ml/Minute auf eine 0,31 x 25 cm RP-18 Säule gepumpt und mit einem Puffergemisch, bestehend aus 5% HPLC-Puffer II und 95% HPLC-Puffer I äquilibriert. Der Säulendurchfluss wird gesammelt und bezüglich seines Proteingehalts und seiner biologischen IL-2 Aktivität untersucht. Die Säule wird bei Raumtemperatur nach dem in Tabelle 4 beschriebenen Gradientenprofil eluiert. Der Durchfluss wird bei 220 nm Wellenlänge gemessen und 1,5 ml Fraktionen werden gesammelt. Ihr Proteingehalt und ihre biologische IL-2 Aktivität werden bestimmt. Der Peak mit maximaler IL-2 Aktivität eluiert bei ca. 74% HPLC-Puffer II (59% Acetonitril). Die Fraktionen werden bei 4-8°C aufbewahrt und gemäss ihrer spezifischen Aktivität vereint.

## Tabelle 4

### Durchführungsbedingungen für die HPLC (RP-18-Säule) eines typischen Procion-Rot Eluats.

| | |
|---|---|
| Probenmenge: | 60 ml |
| Säulenabmessungen | 0,41 x 25 cm |
| HPLC-Puffer I: | 0,01 M Phosphorsäure, 0,05 M Lithiumchlorid |
| HPLC-Puffer II: | 0,01 M Phosphorsäure, 0,05 M Lithiumchlorid, 80% Acetonitril |
| Durchflussrate: | 1 ml/min |

| | % HPLC-Puffer II | Dauer (Min) |
|---|---|---|
| Aequilibrierung | 5 | 20 |
| Probenmenge | – | 60 |
| Waschen | 5 | 15 |
| Schritt 1, Gradient | 5-35 | 15 |
| Schritt 2, Gradient | 35-85 | 50 |
| Schritt 3, konstant | 85 | 5 |
| Schritt 4, konstant | 5 | 5 |

Alle Chromatographieschritte werden bei Raumtemperaturen durchgeführt.

**Patentansprüche**

1. Verfahren zur Herstellung von homogenem, reifem rekombinantem Human-Interleukin-2 (IL-2), dadurch gekennzeichnet, dass man
   (a) Mikroorganismen, die mit einer DNA-Sequenz transformiert sind, die für reifes Human-IL-2 kodiert, kultiviert;
   (b) die transformierten Mikroorganismen zur Expression und Akkumulation von reifem Human-IL-2 veranlasst;
   (c) die Mikroorganismen lysiert; wobei ein Zellysat erhalten wird;
   (d) die im Zellysat enthaltenen Zellmembranbestandteile abtrennt;
   (e) aus den isolierten Zellmembranbestandteilen IL-2 mittels einer Waschlösung, die etwa 4 bis 7M Guanidin-HCl enthält, extrahiert; und
   (f) das IL-2 aus der Waschlösung chromatographisch reinigt.

2. Verfahren nach Anspruch 1, dadurch gekennzeichnet, dass die transformierten Mikroorganismen durch Beschallung lysiert werden.

**3.** Verfahren nach Anspruch 1 oder 2, dadurch gekennzeichnet, dass die Zellmembranbestandteile mittels Zentrifugation vom Zellysat abgetrennt werden.

**4.** Verfahren nach den Ansprüchen 1-3, dadurch gekennzeichnet, dass die isolierten Zellmembranbestandteile vor der Extraktion des IL-2 mit der Waschlösung, die etwa 4 bis 7 M Guanidin-HCl enthält, mit Salz- und Detergenzlösungen gewaschen werden.

**5.** Verfahren nach Anspruch 4, dadurch gekennzeichnet, dass die Waschungen mit den Salz- und Detergenzlösungen in 3 aufeinanderfolgenden, getrennten Verfahrensschritten durchgeführt werden.

**6.** Verfahren nach Anspruch 5, dadurch gekennzeichnet, dass die Zellmembranbestandteile im ersten Verfahrensschritt mit einer NaCl-Lösung, im zweiten Verfahrensschritt mit einer Detergenz- und im dritten Verfahrensschritt mit einer Guanidin-HCl-Lösung mit einer Molarität von ungefähr 1,75 bis 2,0 gewaschen werden.

**7.** Verfahren nach einem der Ansprüche 1-6, dadurch gekennzeichnet, dass die Extraktion des IL-2 aus den isolierten Zellmembranbestandteilen unter Verwendung einer Waschlösung, die ungefähr 7M Guanidin-HCl enthält, erfolgt.

**8.** Verfahren nach Anspruch 1, dadurch gekennzeichnet, dass die chromatographische Reinigung mittels hochauflösender Flüssigkeitschromatographie (HPLC) erfolgt.

**9.** Verfahren nach Anspruch 1, dadurch gekennzeichnet, dass die chromatographische Reinigung mittels Affinitätschromatographie erfolgt.

**10.** Verfahren nach Anspruch 9, dadurch gekennzeichnet, dass es sich bei der Affinitätschromatographie um eine Farbstoff-Affinitätschromatographie handelt.

**11.** Verfahren nach Anspruch 1, dadurch gekennzeichnet, dass es sich bei der chromatographischen Reinigung um ein mehrstufiges Verfahren handelt, bei dem hochauflösende Flüssigkeitschromatographie und Affinitätschromatographie verwendet werden.

**12.** Pharmazeutische Präparate, enthaltend homogenes, reifes, rekombinantes Human-Interleukin-2 (IL-2) mit einer spezifischen Aktivität von mindestens $53 \times 10^6$ E/mg herstellbar nach dem Verfahren gemäss einem der Ansprüche 1 bis 11 als aktiven Bestandteil sowie ein physiologisch verträgliches Trägermaterial.

**Claims**

**1.** A process for the production of homogeneous mature recombinant human interleukin-2 (IL-2), characterized by
    a) cultivating microorganisms transformed with a DNA sequence which codes for mature human IL-2;
    b) causing the transformed microorganisms to express and accumulate mature human IL-2;
    c) lysing the microorganisms to give a cell lysate;
    d) separating the cell membrane components present in the cell lysate;
    e) extracting IL-2 from the isolated cell membrane components using a wash solution which contains about 4 to 7M guanidine HCl; and
    f) chromatographically purifying the IL-2 from the wash solution.

**2.** A process according to claim 1, characterized in that the transformed microorganisms are lysed by sonification.

**3.** A process according to claim 1 or 2, characterized in that the cell membrane components are separated from the cell lysate by centrifugation.

**4.** A process according to any one of claims 1-3, characterized in that the isolated cell membrane components are washed with salt and detergent solutions prior to the extraction of the IL-2 with a wash solution containing about 4 to 7M guanidine HCl.

5. A process according to claim 4, characterized in that the washings with the salt and detergent solutions are carried out in 3 sequential, separate process steps.

6. A process according to claim 5, characterized in that the cell membrane components are washed in the first process step with a NaCl solution, in the second process step with a detergent solution and in the third process step with a guanidine HCl solution having a molarity of approximately 1.75 to 2.0.

7. A process according to any one of claims 1 to 6, characterized in that the extraction of the IL-2 from the isolated cell membrane components is carried out using a wash solution which contains approximately 7M guanidine HCl.

8. A process according to claim 1, characterized in that the chromatographic purification is carried out by high performance liquid chromatography (HPLC).

9. A process according to claim 1, characterized in that the chromatographic purification is carried out by affinity chromatography.

10. A process according to claim 9, characterized in that the affinity chromatography is dye affinity chromatography.

11. A process according to claim 1, characterized in that the chromatographic purification is a multiple step procedure in which high performance liquid chromatography and affinity chromatography are used.

12. A pharmaceutical preparation containing as active ingredient homogeneous, mature, recombinant human interleukin 2 (IL-2) having a specific activity of at least $53 \times 10^6$ U/mg, obtainable in accordance with the process according to any one of claims 1 to 11, and a physiologically compatible carrier material.

**Revendications**

1. Procédé de préparation d'interleukine-2 (IL-2) humaine recombinante mature, homogène, caractérisée en ce que
   (a) on cultive des microorganismes qui sont transformés avec une séquence d'ADN qui code pour l'IL-2 humaine mature;
   (b) on incite les microorganismes transformés à exprimer et à accumuler l'IL-2 humaine mature;
   (c) on lyse les microorganismes; et l'on obtient un lysat cellulaire;
   (d) on sépare les composants de membrane cellulaire contenus dans le lysat cellulaire;
   (e) on extrait l'IL-2 à partir des composants de membrane cellulaire isolés au moyen d'une solution de lavage qui contient du chlorhydrate de guanidine environ 4 à 7 M; et
   (f) on purifie l'IL-2 par chromatographie à partir de la solution de lavage.

2. Procédé selon la revendication 1, caractérisé en ce qu'on lyse les microorganismes transformés par traitement aux ultra-sons.

3. Procédé selon la revendication 1 ou 2, caractérisé en ce qu'on sépare les composants de membrane cellulaire d'avec le lysat cellulaire par centrifugation.

4. Procédé selon les revendications 1-3, caractérisé en ce qu'on lave avec des solutions de sels et de détergents les composants de membrane cellulaire isolés avant l'extraction de l'IL-2 avec la solution de lavage, qui contient du chlorhydrate de guanidine environ 4 à 7 M.

5. Procédé selon la revendication 4, caractérisé en ce que les lavages sont faits avec des solutions de sels et de détergents en trois étapes de procédé successives séparées.

6. Procédé selon la revendication 5, caractérisé en ce qu'on lave les composants de membrane cellulaire

12

dans la première étape du procédé avec une solution de NaCl, dans la seconde étape du procédé avec une solution de détergent et dans la troisième étape du procédé avec une solution de chlorhydrate de guanidine ayant une molarité d'environ 1,75 à 2,0.

7. Procédé selon l'une des revendication 1-6, caractérisé en ce que l'extraction de l'IL-2 s'effectue à partir des composants de membrane cellulaire isolés avec utilisation d'une solution de lavage qui contient du chlorhydrate de guanidine environ 7 M.

8. Procédé selon la revendication 1, caractérisé en ce que la purification chromatographique s'effectue au moyen d'une chromatographie en phase liquide à haute performance (HPLC).

9. Procédé selon la revendication 1, caractérisé en ce que la purification chromatographique se fait par chromatographie d'affinité.

10. Procédé selon la revendication 9, caractérisé en ce qu'il s'agit pour la chromatographie d'affinité d'une chromatographie d'affinité avec colorant.

11. Procédé selon la revendication 1, caractérisé en ce qu'il s'agit pour la purification chromatographique, d'un procédé à plusieurs étapes dans lequel on utilise chromatographie en phase liquide à haute performance et chromatographie d'affinité.

12. Préparations pharmaceutiques contenant de l'interleukine-2 humaine (IL-2) recombinante, mature et homogène, ayant une activité spécifique d'au moins $53 \times 10^6$ E/mg que l'on peut préparer selon le procédé de l'une des revendications 1 à 11 comme composant actif, ainsi qu'un support physiologiquement acceptable.

```
                 10          20          30                          46
 - ACTACTCACA GTAAACTCAA CTCCTGCCAC A ATG TAC AGG ATG CAA CTC
                                      MET Tyr Arg MET Gln Leu
                     61                      76                      91
   CTG TCT TGC ATT GCA CTA AGT CTT GCA CTT GTC ACA AAC AGT
   Leu Ser Cys Ile Ala Leu Ser Leu Ala Leu Val Thr Asn Ser
                            106                     121
 ▼ GCA CCT ACT TCA AGT TCT ACA AAG AAA ACA CAG CTA CAA CTG
   Ala Pro Thr Ser Ser Ser Thr Lys Lys Thr Gln Leu Gln Leu
   136                     151                     166
   GAG CAT TTA CTG CTG GAT TTA CAG ATG ATT TTG AAT GGA ATT
   Glu His Leu Leu Leu Asp Leu Gln MET Ile Leu Asn Gly Ile
   181                     196                     211
   AAT AAT TAC AAG AAT CCC AAA CTC ACC AGG ATG CTC ACA TTT
   Asn Asn Tyr Lys Asn Pro Lys Leu Thr Arg MET Leu Thr Phe
           226                     241                     256
   AAG TTT TAC ATG CCC AAG AAG GCC ACA GAA CTG AAA CAT CTT
   Lys Phe Tyr MET Pro Lys Lys Ala Thr Glu Leu Lys His Leu
                   271                     286                     301
   CAG TGT CTA GAA GAA GAA CTC AAA CCT CTG GAG GAA GTG CTA
   Gln Cys Leu Glu Glu Glu Leu Lys Pro Leu Glu Glu Val Leu
                           316                     331
   AAT TTA GCT CAA AGC AAA AAC TTT CAC TTA AGA CCC AGG GAC
   Asn Leu Ala Gln Ser Lys Asn Phe His Leu Arg Pro Arg Asp
   346                     361                     376
   TTA ATC AGC AAT ATC AAC GTA ATA GTT CTG GAA CTA AAG GGA
   Leu Ile Ser Asn Ile Asn Val Ile Val Leu Glu Leu Lys Gly
           391                     406                     421
   TCT GAA ACA ACA TTC ATG TGT GAA TAT GCT GAT GAG ACA GCA
   Ser Glu Thr Thr Phe MET Cys Glu Tyr Ala Asp Glu Thr Ala
                   436                     451                     466
   ACC ATT GTA GAA TTT CTG AAC AGA TGG ATT ACC TTT TGT CAA
   Thr Ile Val Glu Phe Leu Asn Arg Trp Ile Thr Phe Cys Gln
                   481                         500         510
   AGC ATC ATC TCA ACA CTG ACT TGATAATTAA GTGCTTCCCA CTTAAA
   Ser Ile Ile Ser Thr Leu Thr
    520         530         540         550         560
   ACAT ATCAGGCCTT CTATTTATTT AAATATTTAA ATTTTATATT TATTGTT

   570         580         590         600         610
   GAA TGTATGGTTT GCTACCTATT GTAACTATTA TTCTTAATCT TAAAACT

   620         630         640         650         660
   ATA AATATGGATC TTTTATGATT CTTTTTGTAA GCCCTAGGGG CTCTAAA

   670         680         690         700         710
   ATG GTTTCACTTA TTTATCCCAA AATATTTATT ATTATGTTGA ATGTTAA

   720         730         740         750         760
   ATA TAGTATCTAT GTAGATTGGT TAGTAAAACT ATTTAATAAA TTTGATA

   770         780         790         800
   AAT ATAAACAAAA AAAAAAAAAA AAAAAAAAAA A
```

Figur 1: Nukleotidsequenz des Plasmids pIL2-2B

## Figur 2

pBR322

+ EcoR I

+ Klenow Pol I      + S1

AATTC —————— GAATT    C —————— G
TTAAG —————— CTTAA    G —————— C

+ Bgl II Linker       + Bgl II Linker
+ Ligase           + Ligase

EcoR I     EcoR I

Bgl II    Pst I    Bgl II      Bgl II    Pst I     Bgl II

+ Bgl II        + Bgl II
+ Pst I         + Pst I

+ Ligase

Transform. von RR1:

Bgl II    EcoR I
— Hind III

Sal I

Pst I —

Amp$^R$    Tet$^R$

pRC2

## Figur 3

P$_L$-250     ‚Modell' RBS

Bg/II    Hae III

TTAAAAATTAAGGAGGAATTCAATT
AATTTTTAATTCCTCCTTAAGTTAA

Ligierung

+ Bg/II
+ Eco RI

Isol. des 265 Bp Fragm.

Ligierung
+
Transformation

pRC2

pRC23

Figur 4

EP 0 147 819 B1

[EcoR V]

BamH I     HgiA I                                    Aha III        [EcoR V]  HgiA I          BamH I

←————————————————— IL-2 —————————————————→  ←— pBR322 —→

asn   ser   ala | pro
—AAC  AGT  GCA| CCT—
—TTG  TC|A CGT  GGA—
      HgiA I

HgiA I, Aha III

EcoR I                    HgiA I                    HgiA I                    Aha III          pRC23
5' AATTCAATTATGAGTGCA                              CCT ———————————————
3'      GTTAATACTC                                 ACGTGGA ———————————                          EcoR I
Synthetische DNA                                                                                EcoR V

T4 DNA Ligase

EcoR I  ┌ HgiA I                                                    Aha III/EcoR V
P_L                                    IL-2

EcoR I              met  ser  ala  pro
—— GAATTCAATT ATG  AGT  GCA  CCT ———
—— CTTAAGTTAA TAC  TCA  CGT  GGA ———

**Figur 5**

```
        Ser   Ala | Pro
───── AGT  GCA | CCT ─────
───── TC|A  CGT  GGA ─────
        HgiA I
```

↓ + HgiA I

↓ + T₄ DNA Polymerase

```
               CCT ─────
               GGA ─────
```

λ 108 bp

```
Bgl II        Hae III
───── ───── AGG
───── ───── TCC
```

↓ + T₄ DNA Ligase

↓ + Bgl II
  + Xba I

+Ligase

Transform. von RR1:

pRC 23/IL 2, #4-1

pRC 201/IL 2

## Figur 6

pRC23

pRC201/IL2

+ EcoR I

+ synth. Oligonucleotide :

5'AATTCAATTATGGCT 3'
3'GTTAATACCGA 5'

+Ligase

+ Pst I

Gelreinigung
des 1025 Bp Fragments

+ Pst I

+ Stu I (partial)

Gelreinigung des 3875 Bp
Fragments

+Ligase

Transform. v. RR1 (pRK248clts):

pRC233/IL2